(19) Europäisches Patentamt
European Patent Office
Office européen des brevets

(11) EP 1 663 400 B1

(12) **FASCICULE DE BREVET EUROPEEN**

(45) Date de publication et mention de la délivrance du brevet:
**28.04.2010 Bulletin 2010/17**

(21) Numéro de dépôt: **04767358.7**

(22) Date de dépôt: **16.06.2004**

(51) Int Cl.:
***A61Q 5/10*** *(2006.01)* ***A61K 8/49*** *(2006.01)*

(86) Numéro de dépôt international:
**PCT/FR2004/001496**

(87) Numéro de publication internationale:
**WO 2004/112737 (29.12.2004 Gazette 2004/53)**

(54) **COMPOSITION TINCTORIALE COMPRENANT AU MOINS UN COLORANT DIRECT A CHROMOPHORES MIXTES**

FÄRBEZUSAMMENSETZUNG MIT MINDESTENS EINEM DIREKTEN FARBSTOFF MIT GEMISCHTEN CHROMOPHOREN

TINCTORIAL COMPOSITION COMPRISING AT LEAST ONE DIRECT DYE WITH MIXED CHROMOPHORES

(84) Etats contractants désignés:
**AT BE BG CH CY CZ DE DK EE ES FI FR GB GR HU IE IT LI LU MC NL PL PT RO SE SI SK TR**

(30) Priorité: **16.06.2003 FR 0307185**

(43) Date de publication de la demande:
**07.06.2006 Bulletin 2006/23**

(73) Titulaire: **L'Oréal**
**75008 Paris (FR)**

(72) Inventeurs:
- **SAMAIN, Henri**
  **F-91570 Bièvres (FR)**
- **PLOS, Grégory**
  **Tokyo 158-0097 (JP)**
- **HERCOUET, Leila**
  **F-93360 Neuilly Plaisance (FR)**
- **GREAVES, Andrew**
  **F-77144 Montevrain (FR)**

(74) Mandataire: **Fevrier, Murielle Françoise E.**
**L'Oréal**
**D.I.P.I.**
**25-29 Quai Aulagnier**
**92600 Asnières (FR)**

(56) Documents cités:
**WO-A-03/006554**
**FR-A- 2 825 625**
**US-A- 5 708 151**
**US-A1- 2003 066 143**
**US-B1- 6 530 959**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 663 400 B1

## Description

L'invention a pour objet une composition tinctoriale comprenant un colorant direct constitué de chromophores différents. L'invention a aussi pour objet le procédé de teinture mettant en oeuvre cette composition ainsi que l'utilisation de cette composition pour la teinture des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues en associant ces bases d'oxydation à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

Ce procédé de coloration d'oxydation consiste à appliquer sur les fibres kératiniques, des bases ou un mélange de bases et de coupleurs avec de l'eau oxygénée à titre d'agent oxydant, à laisser pauser, puis à rincer les fibres. Les colorations qui en résultent sont permanentes, puissantes, et résistantes aux agents extérieurs, notamment à la lumière, aux intempéries, aux lavages, à la transpiration et aux frottements. Généralement appliquées à pH basique, il permet d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est à dire de la rendre plus visible.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à les laisser pauser, puis à rincer les fibres.

Il est connu par exemple d'utiliser des colorants directs du type nitrés benzéniques, des colorants anthraquinoniques, des nitropyridines, des colorants du type azoïque, xanthénique, acridinique, azinique ou triarylméthane.

Certains colorants directs peuvent être combinés avec des agents oxydants ce qui permet d'obtenir un éclaircissement de la fibre au moment de la coloration. Par exemple, la demande de brevet EP 810 851 décrit des compositions tinctoriales contenant des colorants directs comportant au moins un atome d'azote quatemisé du type azoïque ou azométhine, qui peuvent être mélangées extemporanément à pH basique à une composition oxydante.

Les colorations qui résultent de l'utilisation de colorants directs sont des colorations temporaires ou semi-permanentes car la nature des interactions qui lient les colorants directs à la fibre kératinique, et leur désorption de la surface et /ou du coeur de la fibre sont responsables de leur faible puissance tinctoriale et de leur mauvaise tenue aux lavages ou à la transpiration.

Ces colorants directs peuvent être constitués d'un ou plusieurs chromophores, identiques ou différents. Des colorants constitués de plusieurs chromophores sont par exemple décrits dans les documents FR 1 540 423, EP 1 133 975, EP 1 133 976, US 5 708 151, WO 02-078596. Tous ces colorants permettent d'obtenir des couleurs chromatiques, c'est à dire des couleurs très en reflets tels que des oranges crus, jaunes crus, rouges crus, etc.

Pour obtenir une coloration non chromatique, les colorants directs sont utilisés en mélange. En particulier, pour l'obtention de couleurs fondamentales, c'est à dire des couleurs variant du noir au blond naturel en passant par les marrons, on utilise des compositions de teinture comprenant généralement un mélange de colorants directs bleus ou violets et de colorants directs jaunes ou oranges. Le problème rencontré avec ces mélanges est que les différents colorants ne présentent pas la même affinité pour le cheveu ou la même résistance aux agents extérieurs ce qui entraîne au cours du temps une altération de la couleur initiale. Par exemple, après plusieurs shampooings, on peut observer un virage de la coloration vers le violet ou le jaune en fonction de l'affinité de chacun des colorants pour la fibre kératinique.

Le but de la présente invention est de fournir des colorants directs permettant de remédier aux inconvénients des colorants directs existants. En particulier, un des buts de la présente invention est de fournir des colorants directs permettant d'obtenir des nuances fondamentales sans problème de virage de la couleur dans le temps. Un autre but de l'invention est de fournir des colorants directs qui permettent de teindre les fibres kératiniques aussi puissamment que les colorants d'oxydation, qui soient aussi stables qu'eux à la lumière, résistants aux intempéries, aux lavages et à la transpiration, et en outre, suffisamment stables en présence d'agents oxydants et réducteurs pour pouvoir obtenir simultanément un éclaircissement de la fibre.

Ce but est atteint avec la présente invention qui a pour objet une composition tinctoriale comprenant, dans un milieu de teinture approprié, un colorant mixte constitué de plusieurs chromophores reliés entre eux par un bras de liaison, au moins deux de ces chromophores étant différents, les chromophores étant choisis de sorte que le colorant mixte, dans une composition à $4{,}7 \times 10^{-4}$ mol de colorant pour 100 g de composition, conduit dans un test de coloration

standardisé sur une mèche de cheveux à 90 % de cheveux blancs, aux valeurs suivantes dans le système colorimétrique L*a*b*:

si a* ≥ 0 et b* ≥ 0,
alors a* appartient à l'intervalle [0 ; +20] et b* appartient à l'intervalle [0 ; +40]

si a* ≤ 0 et b* ≥ 0,
alors a* appartient à l'intervalle [-27 ; 0] et b* appartient à l'intervalle [0 ; +40]

si a* ≥ 0 et b* ≤ 0,
alors a* appartient à l'intervalle [0 ; +9] et b* appartient à l'intervalle [-10 ; 0]

si a* ≤ 0 et b* ≤ 0,
alors a* appartient à l'intervalle [-14; 0] et b* appartient à l'intervalle [-21 ; 0].

Les colorants impliqués sont définis plus loin.

L'invention a aussi pour objet un procédé de teinture mettant en oeuvre cette composition.

Un autre objet de l'invention est l'utilisation de la composition de la présente invention pour la teinture des fibres kératiniques, en particulier les fibres kératiniques humaines telles que les cheveux, en particulier pour obtenir une bonne résistance aux shampooings.

L'invention a enfin pour objet des colorants mixtes tels que définis précédemment.

La composition de la présente invention permet en effet d'obtenir une coloration des fibres kératiniques dans des nuances fondamentales variant du noir au blond naturel et résistante aux différents agents extérieurs, en particulier les shampooings. Elle permet de plus d'éviter les problèmes de virage de la couleur dans le temps.

La mesure des valeurs de L*a*b* telles que définies dans la présente invention doit être effectuée à partir d'une composition de référence qui contient :

| | |
|---|---|
| Colorant mixte utile pour l'invention | 4,7x $10^{-4}$ mol |
| Alkyl (C8/C10 50/50) polyglucoside (2) en solution aqueuse à 60 % tamponnée commercialisé sous le nom ORAMIX-CG110 par la société SEPPIC | 10 g |
| Alcool benzylique | 10 g |
| Polyéthylène glycol 400 à 8 motifs d'oxyde d'ethylène | 12 g |
| Ammoniaque à 20,5 % | 13 g |
| Eau déminéralisée | qsp 100 g |

La composition de référence est appliquée à température ambiante sur des mèches de cheveux naturel à 90% blancs (T = 23°C ± 3°C). Après 20 min de pause, les mèches sont rincées et séchées. Les valeurs de L*a*b* sont mesurées à l'aide d'un colorimètre CM2002, illuminant D65-10° SCI.

Dans le système L* a* b*, les trois paramètres désignent respectivement l'intensité (L*), la nuance (a*) et la saturation (b*). Selon ce système, plus la valeur de L* est élevée, plus la couleur est claire ou peu intense. Inversement, plus la valeur de L* est faible, plus la couleur est foncée ou très intense. a* et b* indiquent deux axes de couleurs, a* indique l'axe de couleur vert/rouge et b* l'axe de couleur bleu/jaune.

Selon un mode de réalisation préféré, les valeurs de L*a*b du colorant mixte utile dans la présente invention sont telles que :

si a* ≥ 0 et b* ≥ 0,
alors a* appartient à l'intervalle [0 ; +15] et b* appartient à l'intervalle [0 ; +30]

si a* ≤ 0 et b* ≥ 0,
alors a* appartient à l'intervalle [-20 ; 0] et b* appartient à l'intervalle [0 ; +30]

si a* ≥ 0 et b* ≤ 0,
alors a* appartient à l'intervalle [0 ; +6] et b* appartient à l'intervalle [-6 ; 0]

si a* ≤ 0 et b* ≤ 0,

alors a* appartient à l'intervalle [-10; 0] et b* appartient à l'intervalle [-15 ; 0].

De préférence, la chromaticité C* calculée selon la formule ci dessous est inférieure à 20.

$$C^* = \sqrt{(a^*)^2 + (b^*)^2}.$$

Selon la présente invention, on entend par « chromophore » un radical issu d'un colorant, c'est à dire un radical issu d'une molécule absorbant dans le domaine visible du rayonnement (entre 400 et 800 nm).

Au sens de la présente invention, les chromophores sont dits différents lorsqu'ils diffèrent par leur structure chimique. De tels chromophores peuvent être des chromophores issus de familles différentes ou d'une même famille à la condition de présenter des structures chimiques différentes. Par exemple, les chromophores peuvent être choisis dans la famille des colorants azoïques mais différer par la structure chimique des radicaux le constituant.

Les colorants mixtes à utiliser dans les compositions de l'invention sont :

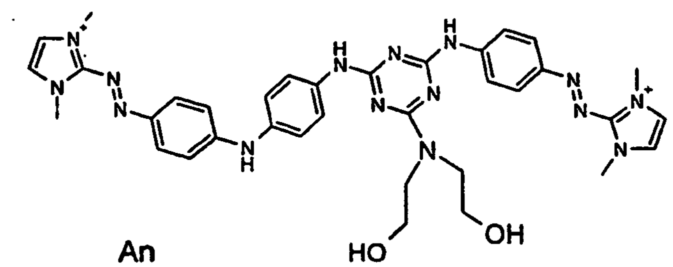

An

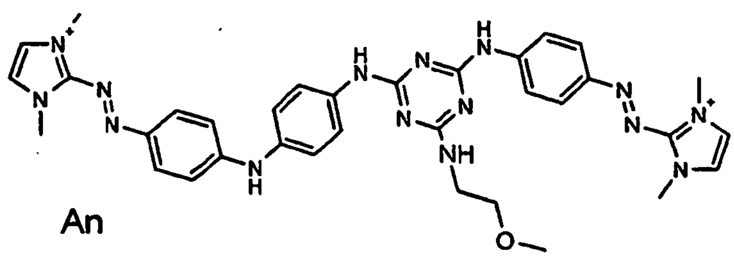

An

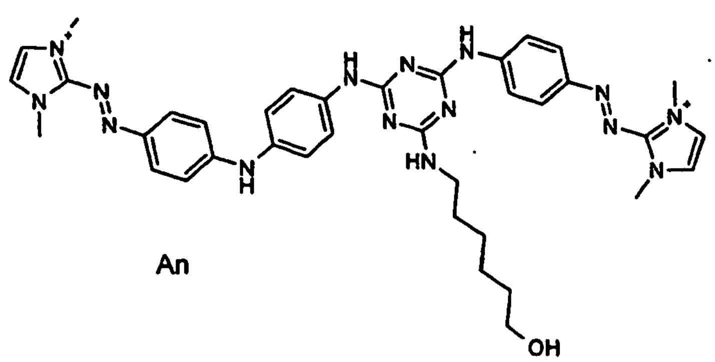

An

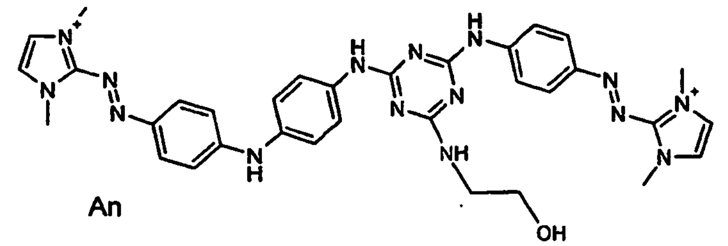

An

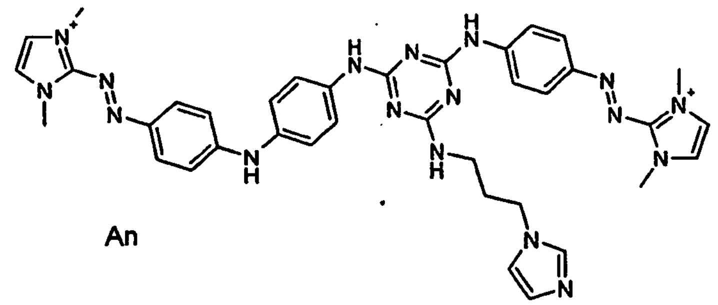

An

On représente un ou plusieurs anions identiques ou différents, monovalents ou polyvalents.

La composition de la présente invention contient en général une quantité de colorant mixte comprise entre 0,001 et 20% par rapport au poids total de la composition. De préférence, cette quantité est comprise entre 0,005 et 10% et encore plus préférentiellement entre 0,01 et 5% par rapport au poids total de la composition.

Les colorants de l'invention peuvent être préparés selon des réactions chimiques connues en soi à partir de chromophores fonctionnalisés capables de réagir avec le bras de liaison choisi. Par exemple, lorsque le bras de liaison est un groupe triazine alors le chromophore doit comporter un groupe réactif amino, OH ou SH et la synthèse peut s'effectuer selon les schémas ci-dessous.

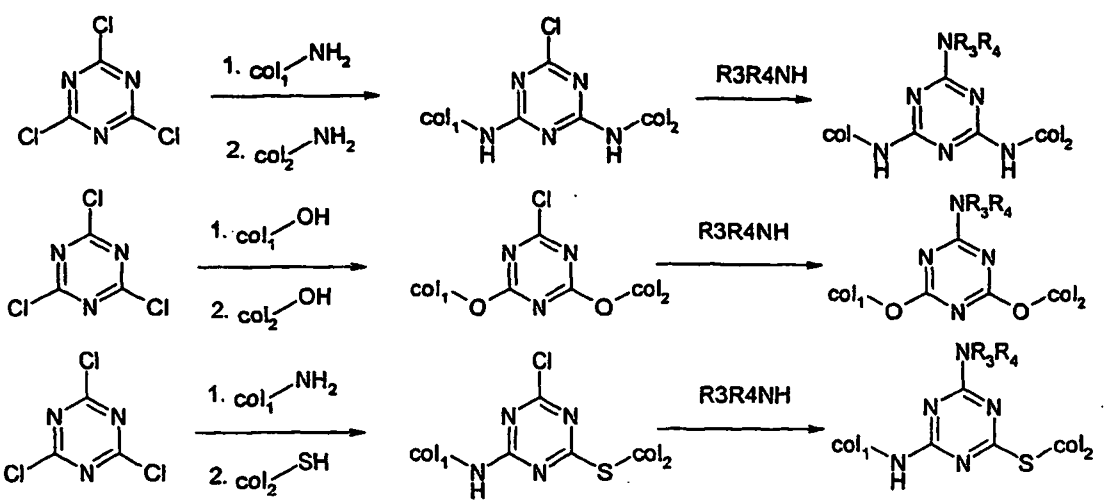


Selon une première étape, un premier chromophore est mélangé au composé formant ou susceptible de former le bras de liaison, par exemple le chlorure de cyanuryle. Lorsque cette réaction est terminée, on ajoute au milieu réactionnel un second chromophore. Cette séquence peut être répétée autant de fois qu'il y a de groupes réactifs sur le composé formant ou susceptible de former le bras de liaison.

Pour la préparation d'un colorant mixte Col1-L-Col 2, le rapport molaire du bras de liaison par rapport au colorant 1 est généralement compris entre 10:1 et 0,5:1, de préférence égal à 1:1. Ce rapport peut être modifié lorsqu'on utilise plus d'un bras de liaison ou plusieurs chromophores.

La température de réaction est en général comprise entre -10˚C et +130˚C, de préférence entre -5˚C et 100˚C. Le temps de réaction dépend de la réactivité des espèces en présence et de la température de réaction. En général, le temps de réaction est compris entre 10 minutes et 8 heures, de préférence entre 30 minutes et 4 heures.

Le pH de la réaction est en général compris entre 3 et 10, de préférence entre 4 et 8.

La réaction peut être conduite dans de l'eau et/ou dans des solvants organiques, seuls ou en mélanges. Plusieurs publications décrivent la réaction d'association chimique entre deux chromophores identiques. On peut citer par exemple les documents ISBN 0901956759, WO 0278596, DE19845640, WO03/029359, US 5 708 151.

En outre, les réactions ou les réactions d'un bras de liaison avec deux composés différents, colorants ou non, ont été décrites dans la littérature, par exemple dans WO03/029359, DE3335956, WO0330909, WO0318021, Journal of Medicinal Chemistry 43(9), 2000, 1892-97 ; Chemiker Zeitung 117(7-8), 1987. 241-5.

La composition tinctoriale conforme à l'invention peut en outre contenir un ou plusieurs colorants directs conventionnellement utilisés dans le domaine de la coloration des fibres kératiniques. A ce titre, on peut notamment citer les colorants nitrés de la série benzénique, les colorants directs azoïques, les colorants directs méthiniques. Ces colorants directs peuvent être de nature non ionique, anionique ou cationique. De préférence, ces colorants directs additionnels sont de nature cationique.

La composition tinctoriale de l'invention peut contenir une ou plusieurs base d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les métaphénylènediamines, les méta-aminophénols, les méta-diphénols, les coupleurs naphtaléniques, les coupleurs hétérocycliques et leur sels d'addition.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La ou les bases d'oxydation présentes dans la composition de l'invention sont en général présentes chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les alkylsulfates comme les méthyl ou éthyl sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

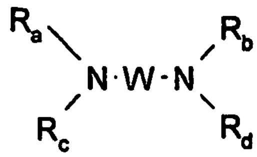
(II)

dans laquelle W est un reste propylène éventuellement substitué par un groupement hydroxyle ou un radical alkyle en $C_1$-$C_4$ ; $R_a$. $R_b$, $R_c$ et $R_d$, identiques ou différents, représentent un atome d'hydrogène, un radical alkyle en $C_1$-$C_4$ ou hydroxyalkyle en $C_1$-$C_4$.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment. Selon un mode de réalisation particulier, la composition de l'invention est appliquée sur les fibres kératiniques en présence d'un agent oxydant pendant un temps suffisant pour obtenir l'éclaircissement souhaité. L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour obtenir l'éclaircissement souhaité. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit' de teinture dans lequel un premier compartiment renferme la composition tinctoriale de la présente invention définie ci-dessus et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention.

## EXEMPLES

## EXEMPLES DE SYNTHESE

### Etape 1 :

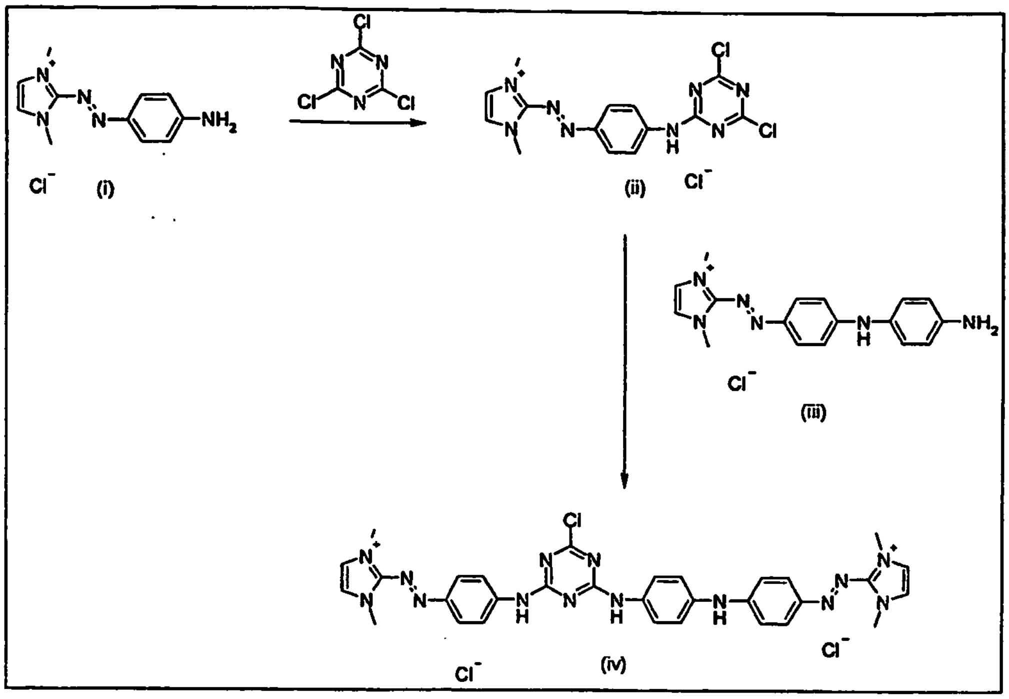

Dans un tricol de 500 ml, on ajoute à température ambiante et sous agitation 2,4 g de chlorure de cyanuryle dans 50 ml d'acétone. Le milieu réactionnel est transparent. On ajoute un mélange constitué d'eau et de glace en proportion respective 50ml et 100 ml et on place le milieu réactionnel à 0°C dans un bain de glace.

Après quelques secondes d'agitation, une suspension blanchâtre est obtenue. La valeur du pH est de 2,8. A l'aide d'une ampoule à brome, on additionne une solution contenant 3,2 g de (i) dissout dans 100 ml d'eau en prenant soin de conserver le pH du mélange réactionnel compris entre 4 et 6 à l'aide d'une solution saturée de $K_2CO_3$ et de garder le milieu réactionnel à une température inférieure à 5°C.

Après cette addition, le pH est stabilisé à 4,8 par ajout régulier de la solution de $K_2CO_3$ saturée. Par la suite, on laisse revenir le mélange à température ambiante. Une HPLC (pureté relative : 94 %) ainsi qu'une masse de contrôle (m/z : 363-365-367) indiquent la formation quasi-exclusive du produit (ii) de réaction

Une solution constituée de 3,42 g de (iii) préalablement dissout dans 50 ml d'un mélange H2O/EtOH (1/1) est ensuite ajoutée au milieu réactionnel précédent en prenant soin de maintenir le pH entre 4 et 6. Après addition, le pH est maintenu à 4,9-5 pendant 15 minutes. Le milieu réactionnel est alors chauffé à 44°C pendant 2,5 heures. On laisse revenir le milieu réactionnel à température ambiante, puis on le verse dans un erlen contenant 1 litre d'une solution constituée d'isopropanol et d'acétone. Un précipité apparaît. Celui-ci est filtré, séché au dessiccateur puis analysé. On obtient 6,08 g (Rdt = 86 %) d'une poudre marron (produit (iv)) d'une pureté égale à 98 % (pureté relative, HPLC).

### Etape 2 :

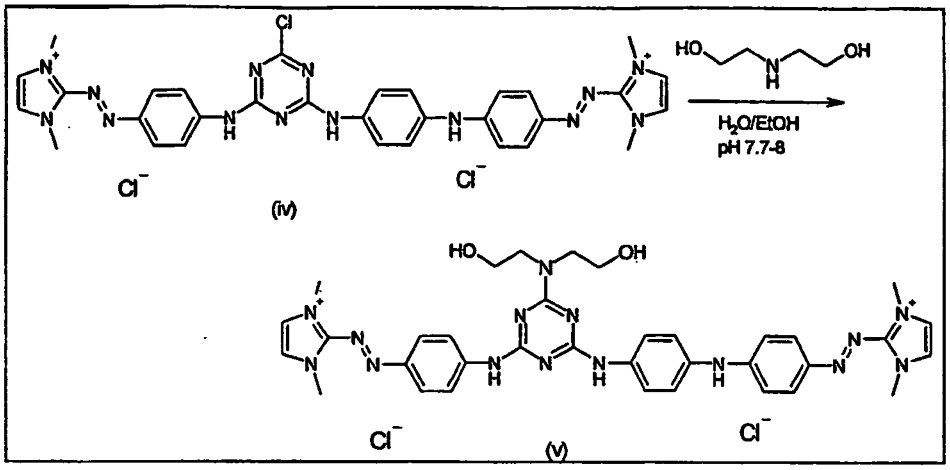


Dans un tricol surmonté d'un réfrigérant, d'une sonde de pH, et d'une ampoule d'addition contenant une solution de soude 1N, on dispose 9 g de (iv) que l'on dissout dans 300 ml d'eau. Le milieu réactionnel est alors chauffé à 85°C (température bain d'huile : 96°C) et on ajuste le pH entre 7,7 et 8 à l'aide de la solution de soude. On additionne alors très lentement l'amine (3 ml) diluée dans 20 ml d'eau en faisant attention de contrôler le pH (pH < 8). Dès que l'addition est terminée, la température du milieu réactionnel est portée à 92°C (température bain d'huile : 118°C) et on laisse sous agitation jusqu'à ce que le pH se stabilise à une valeur comprise entre 7,7 et 8.

Après une heure de réaction, le milieu réactionnel est refroidi puis versé dans un erlen contenant 1 litre d'acétone. Le produit précipite. Il est alors filtré, séché au dessicateur puis analysé. On obtient 7,1 g d'une poudre marron (composé v) (Rdt = 82 %, pureté HPLC : 92 %).
Masse (ESI +) : m/z = 352

RMN ($^1$H, 400 MHz, MeOD) : 3.86-3.87 ppm (s élargi, 4H), 4.05 ppm (s, 6H), 4.12 ppm (s, 6H), 4.88 ppm (s élargi, 4H), 7.10-7.12 ppm (d, 2H), 7.28-7.26 ppm (d, 2H), 7.55 ppm (s, 2H), 7.69 ppm (s, 2H), 7.77-7.79 ppm (d, 2H), 7.97-7.95 ppm (d, 2H), 8.09-8.04 ppm (m, 4H).

Les composés suivants ont été synthétisés en mettant en oeuvre l'étape 2 à partir des réactifs indiqués ci dessous

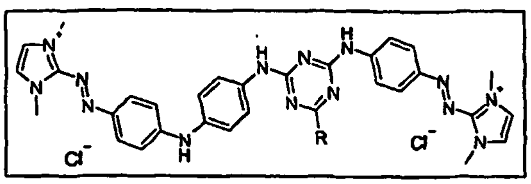

| Structure de R | Réactif utilisé | Masse (ESI +) m/z | pureté HPLC (%) | RdT (%) |
|---|---|---|---|---|
| 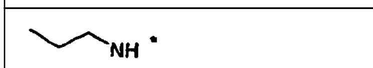 | 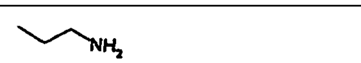 | 329 | 95 | 48 |
| 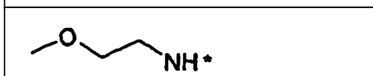 | 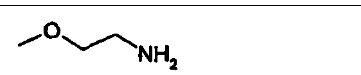 | 337 | 97 | 73 |
| 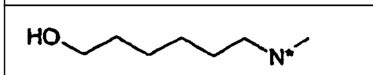 | 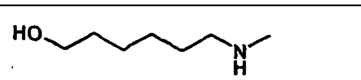 | 358 | 97 | 61 |
| 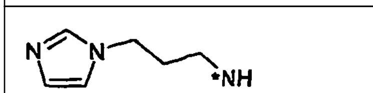 | 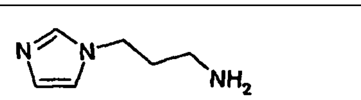 | 362 | 97 | 82 |
| 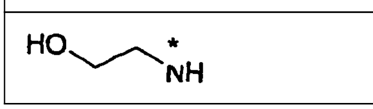 | 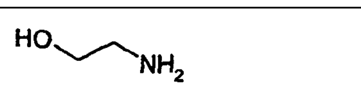 | 330 | 98 | 94 |

(suite)

| Structure de R | Réactif utilisé | Masse (ESI +) m/z | pureté HPLC (%) | RdT (%) |
|---|---|---|---|---|
| 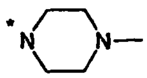 | 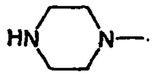 | 349 | 98 | 71 |
| 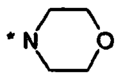 | 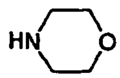 | 342 | 95 | 56 |
| 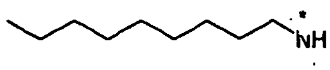 | 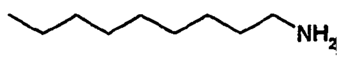 | 371 | 74 | 66 |

**EXEMPLES DE TEINTURE**

**EXEMPLES INVENTION**

On a réalisé les compositions de teinture suivante :

| | |
|---|---|
| Alkyl (C8/C10 50/50) polyglucoside en solution aqueuse à 60 % tamponnée commercialisé sous le nom ORAMIX-CG110 par la société SEPPIC | 10 g |
| Alcool benzylique | 10 g |
| Polyéthylène glycol 400 à 8 motifs d'oxyde d'éthylène | 12 g |
| Colorant mixte cationique (1) | 4,7x $10^{-4}$ mol. |
| Ammoniaque à 20.5 % | 13 g |
| Eau déminéralisée | qsp 100 g |

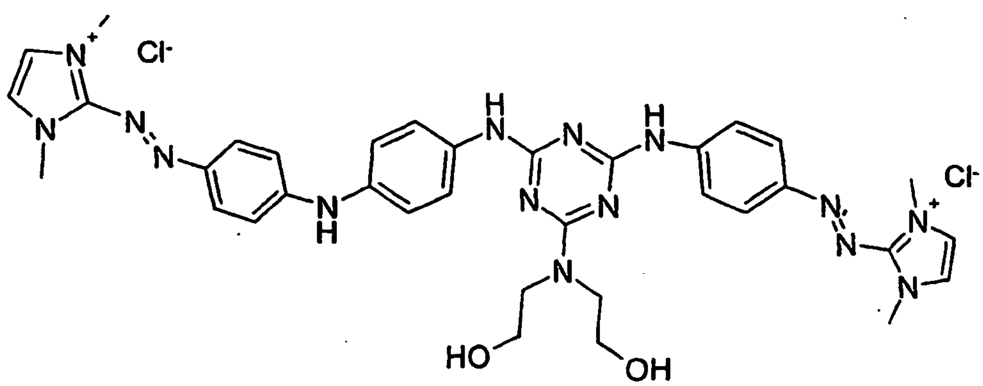

(I)

Au moment de l'emploi, la composition ci dessus est mélangée avec de l'eau oxygénée 40V (poids pour poids). Le mélange est ensuite appliqué sur des mèches de cheveux à 90% blancs naturels (BN) ou permanentés (BP). Le temps de pause sur mèches est de 20 min à température ambiante. Les mèches subissent ensuite un lavage au shampooing.

Après séchage, la montée de couleur est évaluée visuellement et par mesure du L*a*b* (colorimètre CM2002, illuminant D65-10˚ CSI).

La sélectivité est évaluée par la différence entre le niveau de coloration d'une mèche naturelle (BN) et d'une mèche permanentée (BP) à partir des valeurs de L*a*b* mesurées pour chaque type de mèche selon la formule suivante :

$$\Delta E_{bn/bp} = \sqrt{(L_{bp}^* - L_{bn}^*)^2 + (a_{bp}^* - a_{bn}^*)^2 + (b_{bp}^* - b_{bn}^*)^2}.$$

La chromaticité C* est calculée à partir de la formule suivante dans laquelle

$$C^* = \sqrt{(a^*)^2 + (b^*)^2}$$

Les résultats sont regroupés dans le tableau 1 ci dessous

Les mèches colorées subissent ensuite 12 shampooings, avec un séchage intermédiaire entre deux shampooings. La couleur après les 12 shampooings est comparée à la couleur initiale de la mèche colorée, visuellement et par mesure colorimétrique. La résistance aux shampooings est mesurée sur cheveux naturels colorés et sur cheveux permanentés colorés selon la formule de ΔE ci dessus à partir des valeurs de L*a*b* mesurées sur chaque type de mèche avant et après les 12 shampooings.

Les résultats colorimétriques sont regroupés dans le tableau 1 ci dessous.

TABLEAU 1

| | L* | a* | b* | C* | Résistance shampooing |
|---|---|---|---|---|---|
| Cheveux naturels (BN) colorés | 27,7 | 5,6 | -0,9 | 5,6 | - |
| Cheveux permanentés (BP)colorés | 24,15 | 4,9 | -0,5 | 4,9 | - |
| Cheveux naturels (BN) colorés après 12 shampooings | 28,2 | 5,5 | -1,65 | 5,7 | **0,9** |
| Cheveux permanentés (BP) colorés après 12 shampooings | 22,2 | 4,3 | -1,8 | 4,7 | **2,1** |

Les résultats indiqués dans le tableau 1 montrent que la couleur obtenue est puissante et peu chromatique (noir violine). Elles présentent de plus une bonne résistance aux shampooings aussi bien sur cheveux naturels que sur cheveux permanentes. En outre, on n'observe pas de virage de couleur.

## EXEMPLES COMPARATIFS

On a réalisé la composition de teinture suivante :

| | |
|---|---|
| Alkyl (C8/C10 50150) polyglucoside en solution aqueuse à 60 % tamponnée | 10g |
| Alcool benzylique | 10 g |
| Polyéthylène glycol 400 à 8 motifs d'oxyde d'éthylène | 12 g |
| Colorant violet (II) | $4,7 \times 10^{-4}$ mol. |
| Colorant orange (III) | $4,7 \times 10^{-4}$ mol. |
| Ammoniaque à 20.5 % | 13 g |
| Eau déminéralisée | qsp 100 g |

| (II) | (III) |
|---|---|
| 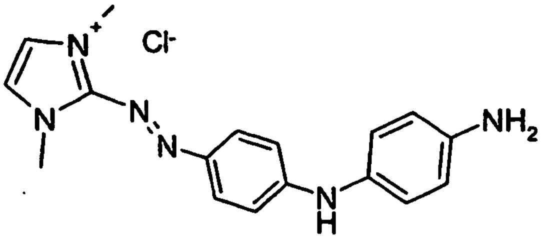 colorant violet | 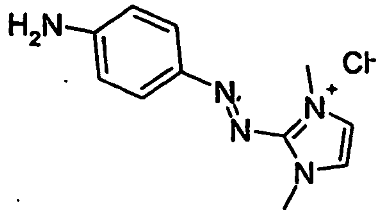 colorant orange |

Le colorant orange est commercialisé sous le nom Basic orange 31. Le colorant violet est synthétisé selon la méthode décrite dans US 5 708 151.

Au moment de l'emploi, la composition est mélangée avec de l'eau oxygénée 40V (poids pour poids). Le mélange est ensuite appliqué sur une mèche de cheveux à 90% blancs, naturels (BN) ou permanentés (BP). Le temps de pause sur mèches est de 20 min à température ambiante. Les mèches subissent ensuite un lavage au shampooing.

Après séchage, la montée de couleur est évaluée visuellement et par mesure colorimétrique L*a*b* telle que définie ci dessus (colorimètre CM2002, illuminant D65-10° CSI).

Les mèches colorées subissent ensuite 12 shampooings, avec un séchage intermédiaire entre deux shampooings. La couleur après les 12 shampooings est comparée à la couleur initiale de la mèche colorée, visuellement et par mesure colorimétrique.

La sélectivité et la résistance aux shampooings sont déterminées selon la méthode décrite précédemment. Les résultats sont regroupés dans le tableau 2 ci dessous.

TABLEAU 2

| | L* | a* | b* | C* | Résistance shampooing |
|---|---|---|---|---|---|
| Cheveux naturels (BN) colorés | 19,4 | 6,4 | -0,4 | 6,4 | |
| Cheveux permanentés (BP)colorés | 17,4 | 1,85 | -0,15 | 1,9 | |
| Cheveux naturels (BN) colorés après 12 shampooings | 20,6 | 9,0 | -3,2 | 9,6 | **4,1** |
| Cheveux permanentés (BP) colorés après 12 shampooings | 18,7 | 8,0 | -7,3 | 10,85 | **9,5** |

Les résultats de la coloration issue du mélange des colorants violet et orange montrent que bien que la couleur obtenue soit peu chromatique, elle varie en fonction de la nature du cheveu qui est coloré. En effet, en comparant les valeurs de a*, la couleur obtenue sur cheveux naturels est rouge alors que sur cheveux permanentés, elle est noire. De plus, un virage de la couleur vers le violet est observé aussi bien sur cheveu naturel que sur cheveu permanenté.

La comparaison des résultats du tableau 1 et du tableau 2 montrent que la présente invention permet d'obtenir des colorations uniformes quelle que soit la nature des cheveux colorés, sans virage et qui présentent une bonne résistance aux shampooings.

**Revendications**

1. Composition tinctoriale comprenant, dans un milieu de teinture approprié, un colorant mixte comprenant plusieurs chromophores reliés entre eux par un bras de liaison, au moins deux de ces chromophores étant différents, les chromophores étant choisis de sorte que le colorant mixte, à partir d'une composition à $4,7x10^{-4}$ mol de colorant pour 100 g de composition, conduit dans un test de coloration standardisé sur une mèche de cheveux à. 90 % da cheveux blancs, aux valeurs suivantes dans le système colorimétrique L*a*b*:

   si a* ≥ 0 et b* ≥ 0,
   alors a* appartient à l'intervalle [0 ; +20] et b* appartient à l'intervalle [0 ; +40]

si a* ≤ 0 et b* ≥ 0,
alors a* appartient à l'intervalle [-27 ; 0] et b* appartient à l'intervalle [0 ; +40]
si a* ≥ 0 et b* ≤ 0,
alors a* appartient à l'intervalle [0 ; +9] et b* appartient à l'intervalle [-10 ; 0]
si a* ≤ 0 et b* ≤ 0,
alors a* appartient à l'intervalle [-14; 0] et b* appartient à l'intervalle [-21 ; 0]

le colorant mixte correspondant à la structure suivante:

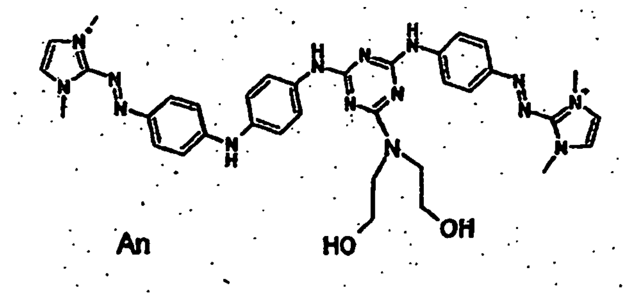

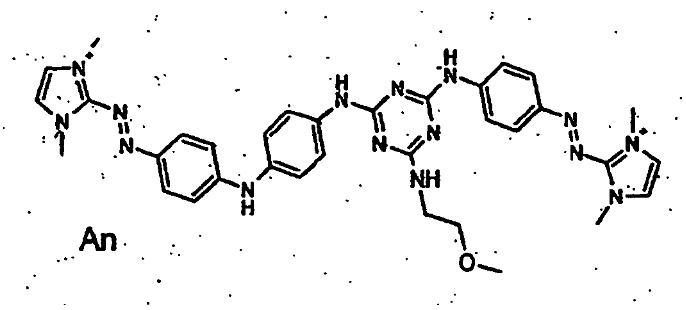

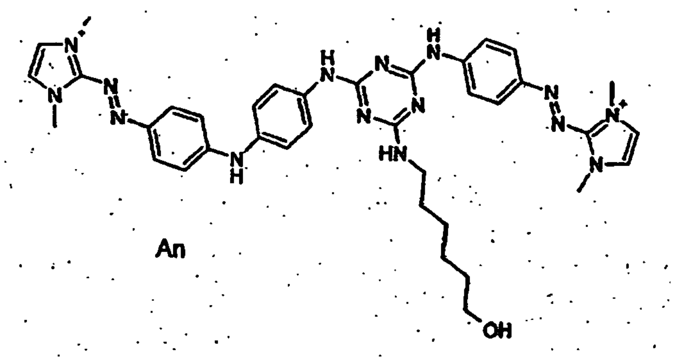

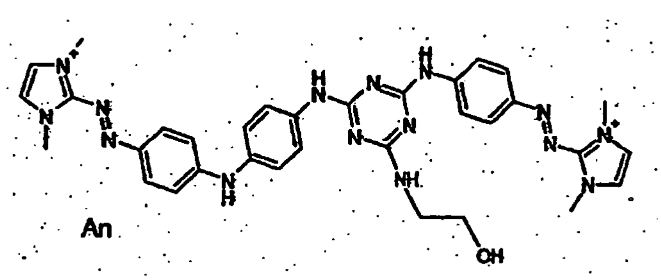

An représente un ou plusieurs anions identiques ou différents, monovalents ou polyvalents.

**2.** Composition selon la revendication 1 dans laquelle les valeurs a* et b* sont telles que
si a* ≥ 0 et b* ≥ 0,

alors a* appartient à l'intervalle [0 ; +15] et b* appartient à l'intervalle [0 ; +30]
si a* ≤ 0 et b* ≥ 0,
alors a* appartient à l'intervalle [-20; 0] et b* appartient à l'intervalle [0; +30]
si a* ≥ 0 et b* ≤ 0,
alors a* appartient à l'intervalle [0 ; +6] et b* appartient à l'intervalle [-6 ; 0]
si a* ≤ 0 et b* ≤ 0,
alors a* appartient à l'intervalle [-10; 0] et b* appartient à l'intervalle [-15; 0].

**3.** Composition selon la revendication 1 ou 2 dans laquelle la chromaticité du colorant est inférieure à 20.

**4.** Composition selon l'une quelconque des revendications précédentes dans laquelle le bras de liaison comprend un atome ou un groupe d'atomes séparant les chromophores du colorant mixte, qui sont tels que la position sur l'échelle des longueurs d'onde du ou des maxima d'absorption des chromophores constituant le colorant mixte ne doit pas être modifiée de plus de 30 manomètres par rapport au maxima d'absotption de chacun des chromophores pris séparément.

**5.** Composition selon l'une quelconque des revendications 1 à 4, **caractérisée en ce que** l'anion An est un anion organique ou minéral choisi parmi les halogénures tels que chlorures, bromures, fluorures, iodures ; les hydroxyde ; les sulfates; les hydrogénosulfates; les alkyl($C_1$-$C_6$)sulfates tels que par exemple le méthylsulfate ou l'éthylsulfate ; les phosphates ; les carbonates ; les hydrogénocarbonates ; les perchlorates ; les acétates ; les tartrates ; les citrates, les oxalates ; les alkyl($C_1$-$C_6$)sulfonates tels que le méthylsulfonate ; les arylsulfonates substitués ou non par un radical alkyle en $C_1$-$C_4$ tels que par exemple un 4-toluylsulfonate.

**6.** Composition selon l'une quelconque des revendications 1 à 5 dans laquelle la quantité de colorant mixte est comprise entre 0,001 et 20%, de préférence, entre 0,005 et 10% et encore plus préférentiellement entre 0,01 et 5% par rapport au poids total de la composition.

**7.** Composition selon l'une quelconque des revendications précédentes comprenant de plus un ou plusieurs colorants directs autres que le colorant mixte tel que défini dans les revendications 1 à 4.

**8.** Composition selon l'une quelconque des revendications précédentes comprenant une base d'oxydation choisie parmi tes paraphénylènediamines, les bis-phénylalkylènediamines, les para-aminophénols, les ortho-aminophénols, les bases hétérocycliques et leurs sels d'addition.

**9.** Composition selon l'une quelconque des revendications précédentes comprenant un coupleur choisi parmi les métaphénylènediamines, les métra-aminophénols, les métadiphénols, les coupleurs naphtaléniques, les coupleurs hétérocycliques et leurs sels d'addition.

**10.** Composition selon la revendication 4 dans laquelle la quantité de chacune des bases d'oxydation est comprise entre 0,001 à 10 % en poids environ du poids total de la composition tinctoriale et la quantité de coupleurs est comprise entre 0,001 et 10 % en poids environ du poids total de la composition tinctoriale.

**11.** Composition selon l'une quelconque des revendications précédentes dans laquelle le pH est compris entre 3 et 12, de préférence compris entre 5 et 11.

**12.** Composition selon l'une quelconque des revendications 1 à 9 comprenant un agent alcalin.

**13.** Composition selon l'une quelconque des revendications précédentes comprenant de plus un agent oxydant.

**14.** Procédé de teinture d'oxydation des fibres kératiniques, **caractérisé en ce qu'**on applique sur les fibres une composition tinctoriale telle que définie dans l'une quelconque des revendications 1 à 10 pendant un temps suffisant pour développer la coloration désirée.

**15.** Procédé selon la revendication 14 dans lequel la composition est appliquée sur les fibres en présence d'un agent oxydant.

**16.** Procédé selon la revendication 15 dans lequel l'agent oxydant est choisi parmi le peroxyde d'hydrogène, le peroxyde d'urée, les bromates de métaux alcalins, les persels, les peracides et les enzymes oxydases.

**17.** Dispositif à plusieurs compartiments dans lequel un premier compartiment contient une composition tinctoriale telle que définie à l'une quelconque dès revendications 1 à 12 et un deuxième compartiment contient un agent oxydant.

**18.** Utilisation de la composition définie aux revendications 1 à 11 pour la teinture de fibres kératiniques.

**19.** Utilisation selon la revendication 16 pour obtenir une bonne résistance aux shampooings.

**20.** Colorant tel que défini à l'une quelconque des revendications 1 à 5.

**21.** Procédé de préparation d'un courant selon la revendication précédente, **caractérisé en ce que** l'on fait réagir un premier chromophore avec un composé formant ou susceptible de former le bras de liaison, puis lorsque cette réaction est terminée, on ajoute au milieu réactionnel un second chromophore ; cette séquence pouvant être répétée autant de fois qu'il y a de groupes réactifs sur le composé formant ou susceptible de former le bras de liaison.

**Claims**

**1.** Dye composition comprising, in a suitable dyeing medium, a mixed dye consisting of several chromophores linked together via a linker, at least two of these chromophores being different, the chromophores being chosen such that the mixed dye, in a composition containing $4.7 \times 10^{-4}$ mol of dye for 100 g of composition, gives, in a standardized coloration test on a lock of hair containing 90% white hairs, the following values in the L*a*b* colorimetric system:

if a* ≥ 0 et b* ≥ 0,
then a* belongs to the range [0; +20] and b* belongs to the range [0; +40]
if a* ≤ 0 et b* ≥ 0,
then a* belongs to the range [-27; 0] and b* belongs to the range [0; +40]
if a* ≥ 0 et b* ≤ 0,
then a* belongs to the range [0; +9] and b* belongs to the range [-10; 0]
if a* ≤ 0 et b* ≤ 0,
then a* belongs to the range [-14; 0] and b* belongs to the range [-21; 0]; the mixed dye corresponding to the following structure:

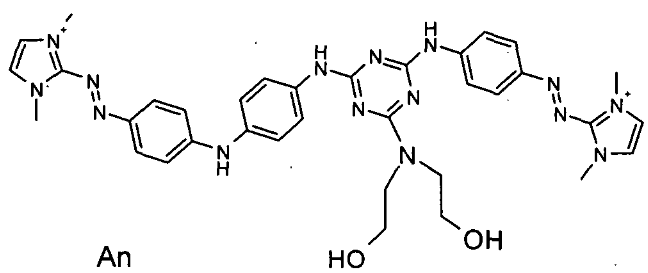

An

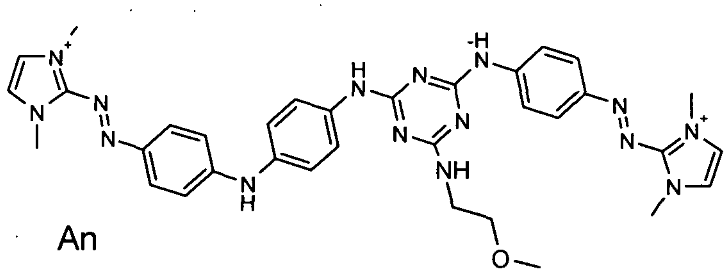

An

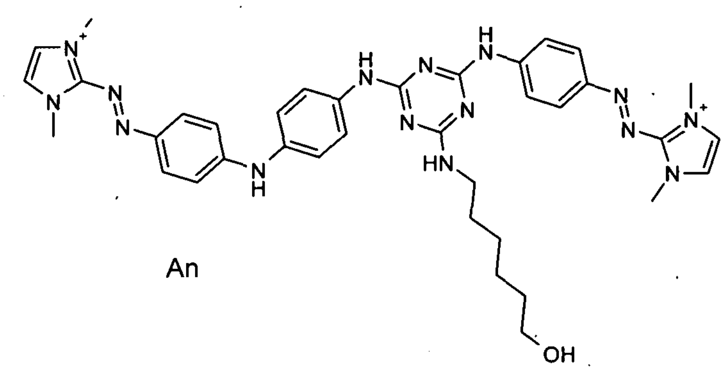

An

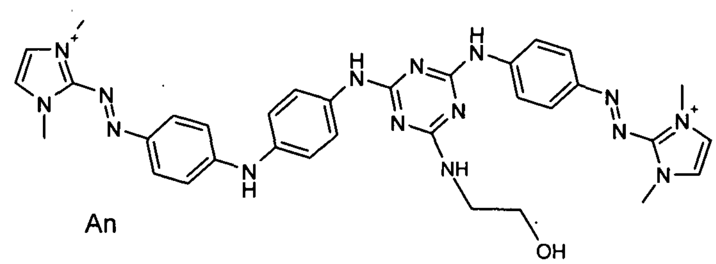

An

An representing one or more identical or different, monovalent or multivalent anions.

**2.** Composition according to Claim 1, in which the values a* and b* are such that:

if a* ≥ 0 et b* ≥ 0,
then a* belongs to the range [0; +15] and b* belongs to the range [0; +30]
if a* ≤ 0 et b* ≥ 0,
then a* belongs to the range [-20; 0] and b* belongs to the range [0; +30]
if a* ≥ 0 et b* ≤ 0,
then a* belongs to the range [0; +6] and b* belongs to the range [-6; 0]
if a* ≤ 0 et b* ≤ 0,
then a* belongs to the range [-10; 0] and b* belongs to the range [-15; 0].

**3.** Composition according to Claim 1 or 2, in which the chromaticity of the dye is less than 20.

**4.** Composition according to any one of the preceding claims, in which the linker comprises an atom or a group of atoms separating the chromophores from the mixed dye, which are such that the position on the scale of wavelengths of the absorption maximum(s) of the chromophores of which the mixed dye is composed should not be modified by more than 30 nm relative to the absorption maxima of each of the chromophores taken separately.

**5.** Composition according to any one of Claims 1 to 4, **characterized in that** the anion An is an organic or mineral anion chosen, for example, from halides such as chlorides, bromides, fluorides or iodides; hydroxides; sulfates; hydrogen sulfates; ($C_1$-$C_6$)alkyl sulfates, for instance methyl sulfate or ethyl sulfate; phosphates; carbonates; hydrogen carbonates; perchlorates; acetates; tartrates; citrates; oxalates; ($C_1$-$C_6$)alkylsulfonates such as methylsulfonate; arylsulfonates optionally substituted with a $C_1$-$C_4$ alkyl radical, for instance a 4-tolylsulfonate.

**6.** Composition according to any one of Claims 1 to 5, in which the amount of mixed dye is between 0.001% and 20%, preferably between 0.005% and 10.% and even more preferably between 0.01% and 5% relative to the total weight of the composition.

**7.** Composition according to any one of the preceding claims, also comprising one or more direct dyes other than the mixed dye as defined in Claims 1 to 4.

**8.** Composition according to any one of the preceding claims, comprising an oxidation base chosen from para-phenylenediamines, bis(phenyl)alkylenediamines, para-aminophenols, ortho-aminophenols and heterocyclic bases, and the addition salts thereof.

**9.** Composition according to any one of the preceding claims, comprising a coupler chosen from meta-phenylenediamines, meta-aminophenols, meta-diphenols, naphthalene-based couplers and heterocyclic couplers, and the addition salts thereof.

**10.** Composition according to Claim 4, in which the amount of each of the oxidation bases is between 0.001% and 10% by weight approximately relative to the total weight of the dye composition, and the amount of couplers is between 0.001% and 10% by weight approximately relative to the total weight of the dye composition.

**11.** Composition according to any one of the preceding claims, in which the pH is between 3 and 12 and preferably between 5 and 11.

**12.** Composition according to any one of Claims 1 to 9, comprising an alkaline agent.

**13.** Composition according to any one of the preceding claims, also comprising an oxidizing agent.

**14.** Process for the oxidation dyeing of keratin fibres, **characterized in that** a dye composition as defined in any one of Claims 1 to 10 is applied to the fibres, for a time that is sufficient to develop the desired coloration.

**15.** Process according to Claim 14, in which the composition is applied to the fibres in the presence of an oxidizing agent.

**16.** Process according to Claim 15, in which the oxidizing agent is chosen from hydrogen peroxide, urea peroxide, alkali metal bromates, persalts, peracids and oxidase enzymes.

**17.** Multi-compartment device in which a first compartment contains a dye composition as defined in any one of Claims 1 to 12 and a second compartment contains an oxidizing agent.

**18.** Use of the composition defined in Claims 1 to 11, for dyeing keratin fibres.

**19.** Use according to Claim 16, to obtain good shampoo fastness.

**20.** Dye as defined in any one of Claims 1 to 5.

**21.** Process for preparing a dye according to the preceding claim **characterized in that** a first chromophore is reacted with a compound forming or capable of forming the linker, and, when this reaction is complete, a second chromophore is then added to the reaction medium; this sequence possibly being repeated as many times as there are reactive groups on the compound forming or capable of forming the linker.

## Patentansprüche

**1.** Farbmittelzusammensetzung, die in einem zum Färben geeigneten Medium einen gemischten Farbstoff enthält, der mehrere Chromophore aufweist, die über eine Verbindungsgruppe aneinander gebunden sind, wobei mindestens zwei dieser Chromophore unterschiedlich sind und
wobei die Chromophore so ausgewählt sind, dass der gemischte Farbstoff, ausgehend von einer Zusammensetzung mit $4{,}7x10^{-4}$ mol Farbstoff auf 100 g Zusammensetzung, in dem colorimetrischen System L*a*b* in einem standardisierten Test an einer Haarsträhne mit 90 % weißen Haaren zu den folgenden Werter:

wenn $a^* \geq 0$ und $b^* \geq 0$,
stammt a* aus dem Intervall [0; +20] und b* stammt aus dem Intervall [0; +40];
wenn $a^* \leq 0$ et $b^* \geq 0$,
stammt a* aus dem Intervall [-27; 0] und b* stammt aus dem Intervall [0; +40];
wenn $a^* \geq 0$ et $b^* \leq 0$,
stammt a* aus dem Intervall [0; +9] und b* stammt aus dem Intervall [-10; 0];
wenn $a^* \leq 0$ et $b^* \leq 0$,

stammt a* aus dem Intervall [-14; 0] und b* stammt aus dem Intervall [-21; 0];

wobei der gemischte Farbstoff der folgenden Struktur entspricht:

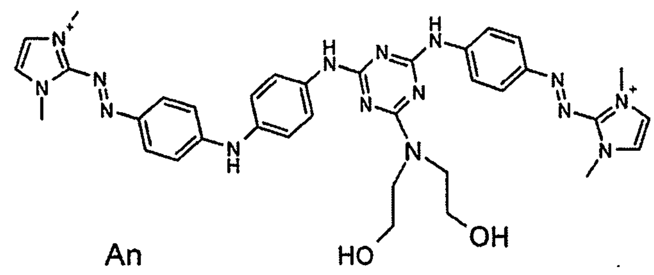

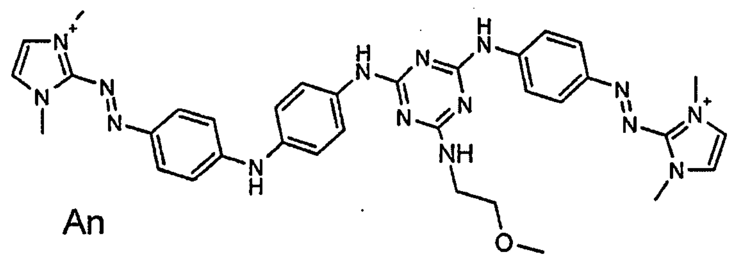

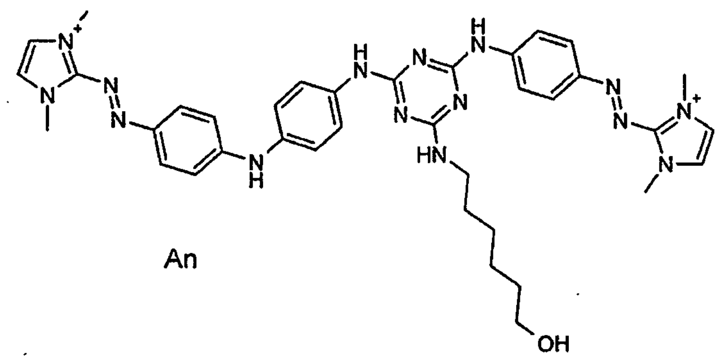

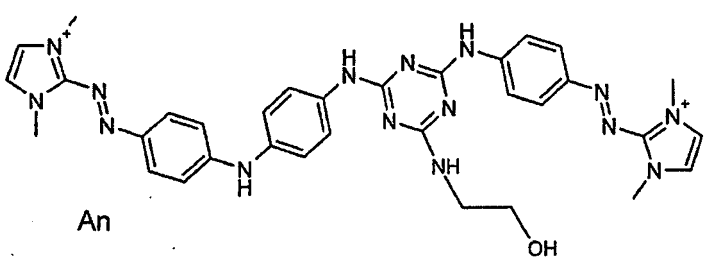

wobei An ein oder mehrere, einwertige oder mehrwertige Anionen bedeutet, die gleich oder verschieden sind.

**2.** Zusammensetzung nach Anspruch 1, wobei die Werte a* und b* so sind, dass
wenn a* ≥ 0 et b* ≥ 0,

stammt a* aus dem Intervall [0; + 15] und b* stammt aus dem Intervall [0; +30];
wenn a* ≤ 0 et b* ≥ 0,
stammt a* aus dem Intervall [-20; 0] und b* stammt aus dem Intervall [0; +30];
wenn a* ≤ 0 et b* ≤ 0,
stammt a* aus dem Intervall [0; +6] und b* stammt aus dem Intervall [-6; 0];
wenn a* ≥ 0 et b* ≤ 0,
stammt a* aus dem Intervall [-10; 0] und b* stammt aus dem Intervall [-15; 0].

**3.** Zusammensetzung nach Anspruch 1 oder 2, wobei die Farbart des Farbstoffes unter 20 liegt.

**4.** Zusammensetzung nach einem der vorhergehenden Ansprüche, wobei die Verbindungsgruppe ein Atom oder eine Gruppe von Atomen aufweist, die die Chromophore des gemischten Farbstoffes trennt, wobei sie so gewählt sind, dass die Position des oder der Absorptionsmaxima der Chromophore, die den gemischten Farbstoff bilden, im Wellenlängenbereich nicht um mehr als 30 Nanometer, bezogen auf das Absorptionsmaximum jedes der Chromophore einzeln, verschoben wird.

**5.** Zusammensetzung nach einem der Ansprüche 1 bis 4, **dadurch gekennzeichnet, dass** das Anion An ein organisches oder anorganisches Anion ist, das unter den Halogeniden, wie Chloriden, Bromiden, Fluoriden, Iodiden; Hydroxiden; Sulfaten; Hydrogensulfaten; Alkyl($C_{1-6}$)sulfaten, wie beispielsweise Methylsulfat der Ethylsulfat; Phosphaten; Carbonaten; Hydrogencarbonaten; Perchloraten; Acetaten; Tartraten; Citraten; Oxalaten; Alkyl($C_{1-6}$)sulfonaten, wie Methylsulfonat; Arylsulfonaten, die gegebenenfalls mit einer $C_{1-4}$-Alkylgruppe substituiert sind, wie beispielsweise 4-Tolylsulfonat, ausgewählt ist.

**6.** Zusammensetzung nach einem der Ansprüche 1 bis 5, wobei der Mengenanteil des gemischten Farbstoffes im Bereich von 0,001 bis 20 %, vorzugsweise 0,005 bis 10 % und noch bevorzugter 0,01 bis 5 %, bezogen auf das Gesamtgewicht der Zusammensetzung, liegt.

**7.** Zusammensetzung nach einem der vorhergehenden Ansprüche, die ferner einen oder mehrere Direktfarbstoffe enthält, die von dem in dem Ansprüchen 1 bis 4 definierten gemischten Farbstoff verschieden sind.

**8.** Zusammensetzung nach einem der vorhergehenden Ansprüche, die eine Oxidationsbase enthält, die unter den p-Phenylendiaminen, Bisphenylalkylendiaminen, p-Aminophenolen, o-Aminophenolen, heterocyclischen Basen und deren Additionssalzen ausgewählt ist.

**9.** Zusammensetzung nach einem der vorhergehenden Ansprüche, die einen Kuppler enthält, der unter den m-Phenylendiaminen, m-Aminophenolen, m-Dihydroxybenzolen, Naphthalinkupplern und heterocyclischen Kupplern und deren Additionssalzen ausgewählt ist.

**10.** Zusammensetzung nach Anspruch 4, bei der der Mengenteil jeder Oxidationsbase im Bereich von etwa 0,001 bis 10 Gew.-% des Gesamtgewichts der Farbmittelzusammensetzung und der Mengenteil der Kuppler im Bereich von etwa 0,001 bis 10 Gew.-% des Gesamtgewichts der Farbmittelzusammensetzung liegt.

**11.** Zusammensetzung nach einem der vorhergehenden Ansprüche, bei der der pH-Wert im Bereich von 3 bis 12 und vorzugsweise 5 bis 11 liegt.

**12.** Zusammensetzung nach einem der Ansprüche 1 bis 9, die ferner einen alkalischen Stoff enthält.

**13.** Zusammensetzung nach einem der vorhergehenden Ansprüche, die ferner ein Oxidationsmittel enthält.

**14.** Verfahren zum oxidativen Färben von Keratinfasern, **dadurch gekennzeichnet, dass** auf die Fasern eine Farbmittelzusammensetzung nach einem der Ansprüche 1 bis 10 während einer Zeitspanne aufgebracht wird, die ausreichend ist, um die gewünschte Farbe zu bilden.

**15.** Verfahren nach Anspruch 14, wobei die Zusammensetzung in Gegenwart eines Oxidationsmittels auf die Fasern aufgebracht wird.

**16.** Verfahren nach Anspruch 15, wobei das Oxidationsmittel unter Wasserstoffperoxid, Harnstoffperoxid, Alkalimetallbromaten, Salzen von Persäuren, Persäuren und Oxidasen ausgewählt ist.

**17.** Vorrichtung mit mehreren Abteilungen, wobei eine erste Abteilung eine Farbmittelzusammensetzung enthält, wie sie in einem der Ansprüche 1 bis 12 definiert ist, und eine zweite Abteilung ein Oxidationsmittel enthält.

**18.** Verwendung der Zusammensetzung nach einem der Ansprüche 1 bis 11 zum Färben von Keratinfasern.

**19.** Verwendung nach Anspruch 16, um eine hohe Beständigkeit gegenüber Haarwäschen zu erhalten.

**20.** Farbstoff, wie er in einem der Ansprüche 1 bis 5 definiert ist.

**21.** Verfahren zur Herstellung eines Farbstoffes nach dem vorhergehenden Anspruch, **dadurch gekennzeichnet, dass** ein erster Chromophor mit einer Verbindung umgesetzt wird, die eine Verbindungsgruppe bildet oder befähigt ist, eine Verbindungsgruppe zu bilden, worauf ein zweiter Chromophor nach Beendigung dieser Reaktion in das Reaktionsmedium gegeben wird; diese Abfolge kann so viele Male wiederholt werden wie reaktive Gruppen an der Verbindung vorhanden sind, die eine Verbindungsgruppe bildet oder befähigt ist, eine Verbindungsgruppe zu bilden.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 810851 A
- FR 1540423
- EP 1133975 A
- EP 1133976 A
- US 5708151 A
- WO 02078596 A
- WO 0278596 A
- DE 19845640
- WO 03029359 A
- DE 3335956
- WO 0330909 A
- WO 0318021 A
- FR 2586913

### Littérature non-brevet citée dans la description

- *Journal of Medicinal Chemistry,* 2000, vol. 43 (9), 1892-97
- *Chemiker Zeitung,* 1987, vol. 117 (7-8), 241-5